# EUROPEAN PATENT SPECIFICATION

(11) **EP 1 299 384 B1**
(45) Date of publication and mention of the grant of the patent: **24.03.2004**
(21) Application number: 01940242.9
(22) Date of filing: 13.06.2001
(51) Int. Cl.: C07D 417/14, A61K 31/38, A61K 31/395, A61K 31/4045, A61K 31/4523, A61K 31/496, A61P 25/06, A61P 25/18, A61P 25/20, A61P 25/22, A61P 25/24, A61P 25/28

(54) **INDOLE DERIVATIVES USEFUL FOR THE TREATMENT OF CNS DISORDERS**
INDOLDERIVATE ZUR VERWENDUNG IN DER BEHANDLUNG VON ZNS-STÖRUNGEN
DERIVES D'INDOLE SERVANT AU TRAITEMENT DES TROUBLES DU SNC

(30) Priority: 19.06.2000 DK 200000957; 20.06.2000 US 212532 P
(43) Date of publication of application: 09.04.2003
(73) Proprietor: H. Lundbeck A/S, 2500 Valby-Copenhagen (DK)
(72) Inventor: BANG-ANDERSEN, Benny, DK-2200 Kobenhavn N (DK); LARSEN, Krestian, DK-4100 Ringsted (DK); KEHLER, Jan, DK-2800 Kgs. Lyngby (DK)
(74) Representative: Nielsen, Leila
(86) International application number: PCT/DK2001/000408
(87) International publication number: WO 2001/098298

(56) References cited:
- EP-A1- 0 722 942
- EP-A1- 0 854 146
- WO-A1-94/20497
- DIRK ROEDA ET AL.: 'Synthesis of (11C) RPR-72840A and its evaluation as a radioligand for the sorotonin reuptake site in positron emission tomography' BIOORGANIC & MEDICINAL CHEMISTRY vol. 5, no. 2, 1997, pages 397 - 403, XP001056777

## Description

### Field of the Invention

The present invention relates to a novel class of indole derivatives having affinity for the dopamine D₄ receptor. The compounds are therefore useful in the treatment of certain psychiatric and neurologic disorders, in particular psychoses. The compounds also have affinity for the 5-HT_{2A} receptor.

### Background of the Invention

Dopamine D₄ receptors belong to the dopamine D₂ subfamily of receptors, which is considered to be responsible for the antipsychotic effects of neuroleptics. The side effects of neuroleptic drugs, which primarily exert their effect via antagonism of D₂ receptors, are known to be due to D₂ receptor antagonism in the striatal regions of the brain. However, dopamine D₄ receptors are primarily located in areas of the brain other than striatum, suggesting that antagonists of the dopamine D₄ receptor will be devoid of extrapyramidal side effects. This is illustrated by the antipsychotic clozapine, which exerts higher affinity for D₄ than D₂ receptors and is lacking extrapyramidal side effects (Van Tol et al. *Nature* **1991**, *350,* 610; Hadley *Medicinal Research Reviews* **1996**, *16*, 507-526, and Sanner *Exp. Opin*. *Ther*. *Patents* **1998**, *8*, 383-393).

A number of D₄ ligands, which were postulated to be selective D₄ receptor antagonists (L-745,879 and U-101958), have been shown to posses antipsychotic potential (Mansbach et al *Psychopharmacology* **1998,** *135,* 194-200). However, recently it has been reported that these compounds are partial D₄ receptor agonists in various *in vitro* efficacy assays (Gazi et al. *Br*. *J*. *Pharmacol*. **1998**, *124*, 889-896 and Gazi et al. *Br*. *J*. *Pharmacol*. **1999**, *128*, 613-620). Furthermore, it was shown that clozapine, which is an effective antipsychotic, is a silent antagonist (Gazi et al. *Br*. *J*. *Pharmacol*. **1999**, *128*, 613-620).

Consequently, D₄ ligands, which are partial D₄ receptor agonists or antagonists, may have beneficial effects against psychoses.

Dopamine D₄ antagonists may also be useful for the treatment of cognitive deficits (Jentsch *et al*. *Psychopharmacology* **1999**, 142:78-84).

Furthermore, evidence for a genetic association between the "primarily inattentive" subtype of attention deficit hyperactivity disorder and a tandem duplication polymorphism in the gene encoding the dopamine D₄ receptor has been published (McCracken et al. *Mol*. *Psychiat*. **2000**, 5, 531-536). This clearly indicates a link between the dopamine D₄ receptor and attention deficit hyperactivity disorder and ligands affecting this receptor may be useful for the treatment of this particular disorder.

Various effects are known with respect to compounds, which are ligands at the different serotonin receptor subtypes. As regards the 5-HT_{2A} receptor, which was previously referred to as the 5-HT₂ receptor, the following effects have been reported e.g.:

Antidepressive effect and improvement of the sleep quality (Meert et al. *Drug*. *Dev*. *Res*. **1989,** *18*, 119.), reduction of the negative symptoms of schizophrenia and of extrapyramidal side-effects caused by treatment with classical neuroleptics in schizophrenic patients (Gelders *British J*. *Psychiatry* **1989**, *155* (suppl. 5), 33). Furthermore, selective 5-HT_{2A} antagonists could be effective in the prophylaxis and treatment of migraine (Scrip Report; "Migraine - Current trends in research and treatment"; PJB Publications Ltd.; May 1991) and in the treatment of anxiety (Colpart et al, *Psychopharmacology* **1985**, *86*, 303-305 and Perregaard et al. *Current Opinion in Therapeutic Patents* **1993**, *1*; 101-128).

Some clinical studies implicate the 5-HT₂ receptor subtype in aggressive behaviour. Furthermore, atypical serotonin-dopamine antagonist neuroleptics, have 5-HT₂ receptor antagonistic effect in addition to their dopamine blocking properties, and have been reported to possess anti-aggressive behaviour (Connor et al. *Exp*. *Opin*. *Ther*. *Patents*. **1998**, *8(4)*, 350-351).

Recently, evidence has also accumulated which support the rational for selective 5-HT_{2A} antagonists as drugs capable of treating positive symptoms of psychosis (Leysen et al. *Current Pharmaceutical Design* **1997**, *3*, 367-390 and Carlsson *Current Opinion in CPNS Investigational Drugs* **2000**, *2*(1), 22-24).
Accordingly, dopamine D₄ receptor ligands are potential drugs for the treatment of schizophrenia and other psychoses, and compounds with combined effects at dopamine D₄ and 5-HT_{2A} receptors may have the further benefit of improved effect on positive and negative symptoms in schizophrenia, including depressive and anxiety symptoms.

Dopamine D₄ ligands related to the compounds of the invention are known from WO 98/28293. The indane and dihydroindole derivatives disclosed herein have the general formula 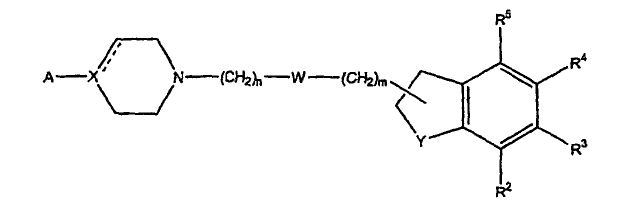 wherein A is an indole and Y is a group completing an indane or a dihydroindole and the other substituents are as defined in the application.

EP 722 942 claim compounds having the formula 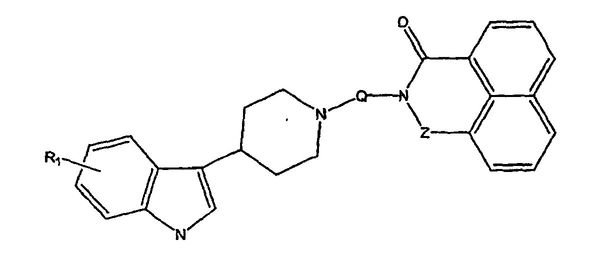 wherein Q is a chain and Z is CO, SO₂ or SO and the other substituents are as defined in the application. The compouds are said to have serotonin agonistic and -antagonistic activities. The compounds are in particular said to bind to 5-HT₂ and D₂ receptors.

Other dopamine D₄ ligands, wherein the indane or dihydroindole is replaced by a pyrrolo[2,3-b]pyridine, a benzimidazole or a furo[2,3-b]pyridine, are described in WO 94/20497, WO 94/22839 and US 5,700,802.

### Summary of the Invention

The object of the present invention is to provide compounds that are partial agonists or antagonists at the dopamine D₄ receptor, in particular such compounds with combined effects at the dopamine D₄ receptors and the 5-HT_{2A} receptor.

A further object of the invention is to provide compounds with no or only low affinity for alpha-1 adrenergic receptors.

Accordingly, the present invention relates to novel compounds of formula I 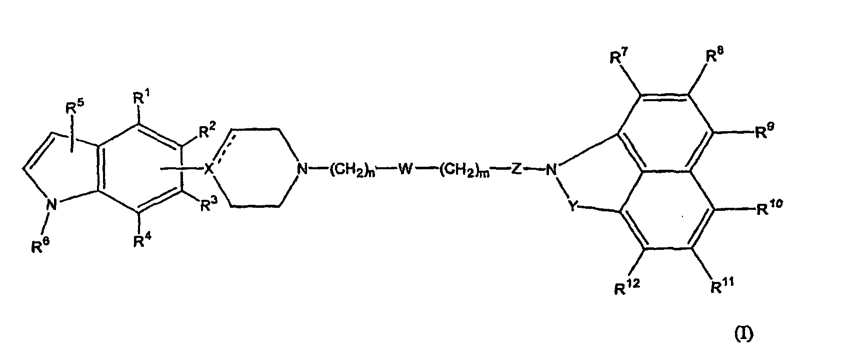 wherein Y is CO, CS, SO, SO₂ or CH₂; Z is CO, CS, SO, SO₂ or CH₂; provided that only one of Y and Z is CO, CS, SO or SO₂;
W is a bond, O, S, CO, CS, SO or SO₂;
n is 0-5, m is 0-5 and n + m is 1-6; provided that when W is O or S, then n ≥ 2 and m ≥ 1, when W is CO, CS, SO or SO₂ then n ≥ 1 and m ≥1;
X is N or CH and the dotted line represents no bond, or X is C and the dotted line represents a bond;
one of R¹, R², R³ and R⁴ forms a bond to X and the others of R¹, R², R³ and R⁴ and R⁵ and R⁷ - R¹² are selected from hydrogen, halogen, cyano, nitro, amino, C₁₋₆-alkyl-amino, di-( C₁₋₆-alkyl )-amino, C₁₋₆-alkyl, C₂₋₆-alkenyl, C₂₋₆-alkynyl, C₁₋₆ alkoxy, C₁₋₆-alkylthio, hydroxy, C₁₋₆-alkyl substituted with hydroxy or thiol, C₃₋₈-cycloalkyl, C₃₋₈-cycloalkyl-C₁₋₆-alkyl, acyl, thioacyl, trifluoromethyl, trifluoromethylsulfonyl and C₁₋₆-alkylsulfonyl; and
R⁶ is hydrogen, C₁₋₆-alkyl, C₂₋₆-alkenyl, C₂₋₆-alkynyl, C₁₋₆-alkyl substituted with hydroxy or thiol, C₃₋₈-cycloalkyl, C₃₋₈-cycloalkyl-C₁₋₆-alkyl, acyl, thioacyl, trifluoromethylsulfonyl and C₁₋₆ alkylsulfonyl;
or pharmaceutically acceptable salts thereof

In a first particular embodiment, the present invention relates to compounds wherein Z is CH₂ and Y is SO₂.

In a second embodiment, the invention relates to such compounds wherein one of R¹, R², R³ and R⁴ forms a bond to X and the others of R¹, R², R³ and R⁴ and R⁵ and R⁷ - R¹² are selected from hydrogen, halogen, cyano, nitro, amino, C₁₋₆-alkyl, C₁₋₆ alkoxy, C₁₋₆-alkylthio, hydroxy, and trifluoromethyl andR⁶ is hydrogen, C₁₋₆-alkyl, or C₁₋₆-alkylcarbonyl.

In a third embodiment, the present invention relates to compounds wherein R² or R³ forms a bond to X.

In a fourth embodiment, the invention relates to such compounds wherein X is N.

In a fifth embodiment, the invention relates to such compounds wherein X is C.

In a sixth embodiment, the invention relates to such compounds wherein X is CH.

In a seventh embodiment, the invention relates to such compounds wherein W is a bond and n + m is 1 to 4, in particular 3.

Specific compounds according to the invention are
2-{3-[4-(1*H*-Indol-5-yl)piperazin-1-yl]propan-1-yl}-2*H*-naphtho[1,8-cd]isothiazole 1,1-dioxide,
2-{3-[4-(1*H*-Indol-6-yl)piperazin-1-yl]propan-1-yl}-2*H*-naphtho[1,8-cd]isothiazole1,1-dioxide,
2-{3-[1-(1*H*-Indol-5-yl)-3,6-dihydro-2*H*-pyridin-4-yl]propan-1-yl}-2*H*-naphtho[1,8-cd]isothiazole 1,1-dioxide
2-{3-[1-(1*H*-Indol-5-yl)piperidin-4-yl]propan-1-yl}-2*H*-naphtho[1,8-cd]isothiazole 1,1-dioxide
or a pharmaceutically acceptable acid addition salt thereof.

The compounds of the invention have been found to show high affinity for dopamine D₄ receptors and to be partial agonists or antagonists at dopamine D₄ receptors. The compounds also show affinity for serotonergic 5-HT_{2A} receptors.
Accordingly, the compounds of the invention are considered useful in the treatment of positive and negative symptoms of schizophrenia and other psychoses, anxiety disorders, such as generalised anxiety disorder, panic disorder, and obsessive compulsive disorder, depression, aggression, cognitive disorders, side effects induced by conventional antipsychotic agents, migraine, attention deficit hyperactivity disorder and in the improvement of sleep.

In another aspect, the present invention provides a pharmaceutical composition comprising at least one compound of formula I as defined above or a pharmaceutically acceptable acid addition salt thereof in a therapeutically effective amount and in combination with one or more pharmaceutically acceptable carriers or diluents.

In a further aspect, the present invention provides the use of a compound of formula I as defined above or an acid addition salt thereof for the manufacture of a pharmaceutical preparation for the treatment of the above mentioned disorders.

### Detailed Description of the Invention

The compounds of general formula I may exist as optical isomers thereof and such optical isomers are also embraced by the invention.

The term C₁₋₆-alkyl refers to a branched or unbranched alkyl group having from one to six carbon atoms inclusive, such as methyl, ethyl, 1-propyl, 2-propyl, 1-butyl, 2-butyl, 2-methyl-2-propyl,2-methyl-1-propyl, pentyl and hexyl.

Similarly, C₂₋₆-alkenyl and C₂₋₆-alkynyl, respectively, designate such groups having from two to six carbon atoms, including one double bond and triple bond respectively, such as ethenyl, propenyl, butenyl, ethynyl, propynyl and butynyl.

The terms C₁₋₆-alkoxy, C₁₋₆-alkylthio, C₁₋₆-alkylsulfonyl, C₁₋₆-alkylamino, C₁₋₆-alkylcarbonyl, etc. designate such groups in which C₁₋₆-alkyl is as defined above.

The term C₃₋₈-cycloalkyl designates a monocyclic or bicyclic carbocycle having three to eight C-atoms, such as cyclopropyl, cyclopentyl, cyclohexyl, etc.

Halogen means fluoro, chloro, bromo or iodo.

As used herein the term acyl refers to a formyl, C₁₋₆-alkylcarbonyl, arylcarbonyl, aryl-C₁₋₆-alkylcarbonyl, C₃₋₈-cycloalkylcarbonyl or a C₃₋₈-cycloalkyl-C₁₋₆-alkyl-carbonyl group and the term thioacyl is the corresponding acyl group in which the carbonyl group is replaced with a thiocarbonyl group.

The term aryl refers to a carbocyclic aromatic group, such as phenyl or naphthyl, in particular phenyl, including methyl substituted phenyl or napthyl.

The acid addition salts of the compounds of the invention are pharmaceutically acceptable salts formed with non-toxic acids. Exemplary of such organic salts are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic and theophylline acetic acids, as well as the 8-halotheophyllines, for example 8-bromotheophylline. Exemplary of such inorganic salts are those with hydrochloric, hydrobromic, sulfuric, sulfamic, phosphoric and nitric acids.

The pharmaceutical compositions of this invention or those which are manufactured in accordance with this invention may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients or other additives normally used in the art may be used.

Conveniently, the compounds of the invention are administered in unit dosage form containing said compounds in an amount of about 0.01 to 100 mg.

The total daily dose is usually in the range of about 0.05 - 500 mg and most preferably about 0.1 to 50 mg of the active compound of the invention.

The compounds of the invention may be prepared as follows:
1) Alkylating a piperazine, piperidine or tetrahydropyridine of formula II with an alkylating derivative of formula III: 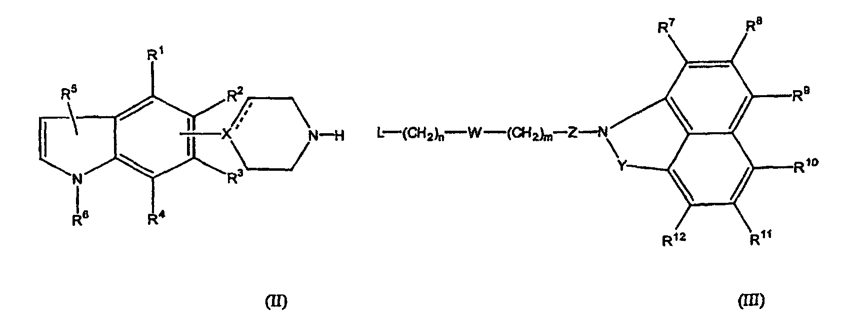 wherein R¹-R¹², X, Z, Y, n, m, W and the dotted line are as previously defined, and L is a leaving group such as e.g. halogen, mesylate or tosylate;
2) Reductive alkylation of an amine of formula II with a reagent of formula IV: 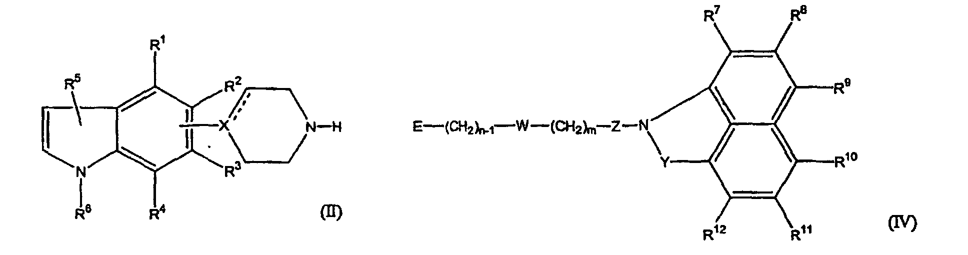 wherein R¹-R¹², X, Z, Y, n, m, W and the dotted line are as previously defined, and E is either an aldehyde or an activated carboxylic acid group;
3) Alkylating a compound of formula VI with an alkylating derivative of formula V: 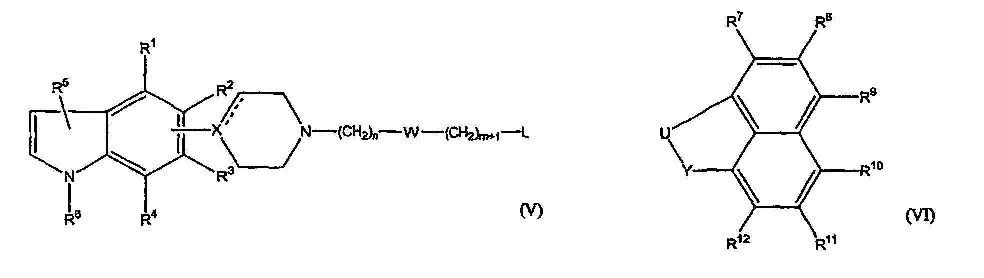 wherein R¹-R¹², X, Y, n, m, W and the dotted line are as previously defined and L is a leaving group such as e.g. halogen, mesylate or tosylate, and U is NH or N⁻ ;
4) Reducing the tetrahydropyridinyl double bond in derivatives of formula VII: 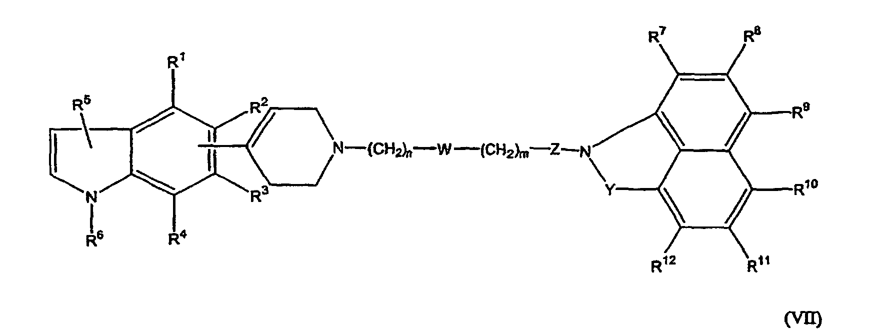 wherein R¹-R¹², Z, Y, n, m and W are as previously defined;
5) Reducing the amide carbonyl in a compound of formula VIII: 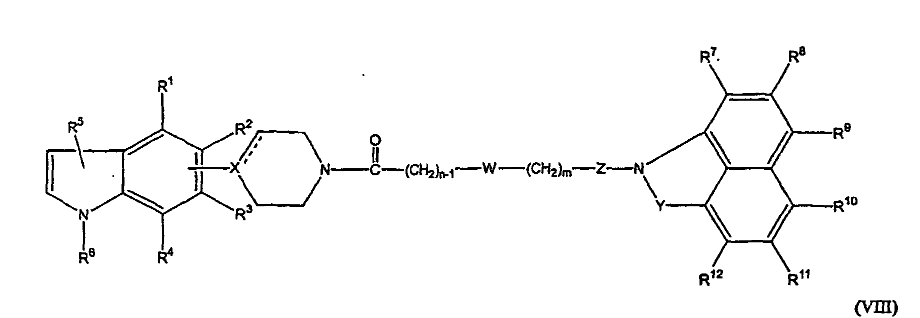 wherein R¹-R¹², X, Y, Z, n, m, W and the dotted line are as previously defined;
6) Reducing the amide carbonyl in a compound of formula IX: 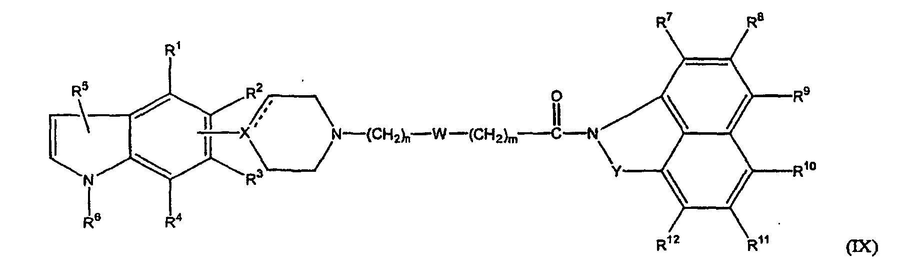 wherein R¹-R¹², X, Y, n, m, W, and the dotted line are as previously defined;
7) Reductive alkylation of an amine of formula XI with a reagent of formula X: 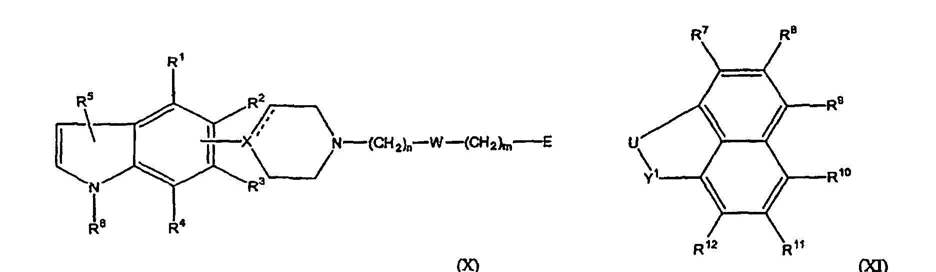 wherein R¹-R¹², X, n, m, W and the dotted line are as previously defined, and E is either an aldehyde or an activated carboxylic acid group, Y¹ is CH₂ and U is NH, or;
8) Acylation of an amine of formula XI with a reagent of formula X: 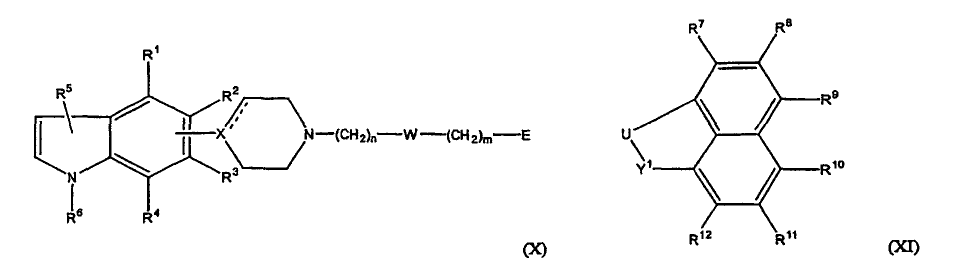
wherein R¹-R¹², X, n, m, W and the dotted line are as previously defined, and E is either an aldehyde or an activated carboxylic acid group, Y¹ is CH₂ , U is NH; whereupon the compound of Formula (I) is isolated as the free base or a pharmaceutically acceptable acid addition salt thereof.

The alkylation according to methods 1) and 3) is conveniently performed in an inert organic solvent such as a suitably boiling alcohol or ketone, preferably in the presence of an organic or inorganic base (potassium carbonate, diisopropylethylamine or triethylamine) at reflux temperature. Alternatively, the alkylation can be performed at a fixed temperature, which is different from the boiling point, in one of the above mentioned solvents or in dimethyl formamide (DMF), dimethylsulfoxide (DMSO) or *N*-methylpyrrolidin-2-one (NMP), preferably in the presence of a base.

Some of the amines of formula (II) are known from the literature or may be prepared analogously (see WO 98/28293, WO 94/20459 and US patent No. 5,576,319). Piperazines of formula (II) may be prepared from nitroindoles by reduction of the nitro group to an aniline, which subsequently is subjected to piperazine synthesis by methods obvious to a chemist skilled in the art (see also Kruse et al. *Red*.*Trav*.*Chim*. Pays.Bas. **1988** *107*, 303-309). Piperidines such as 5-(piperidin-4-yl)-1*H*-indoles may be prepared from the corresponding tetrahydropyridines (WO 94/20459). Alkylating reagents of formula (III) are known from the literature (see Malleron et al. *J*. *Med. Chem*. **1991**, *43*, 2477-2483). Alkylating reagents of formula (V) can be prepared by methods obvious to a chemist skilled in the art, and compounds of formula (VI) are commercially available or described in the literature.

The reductive alkylation according to methods 2) and 7) is performed by standard literature methods. The reaction can be performed in two steps, e.g. coupling of derivatives of formula (II/XI) and the reagent of formula (IV/X) by standard methods via the carboxylic acid chloride or by use of coupling reagents such as e.g. dicyclohexyl carbodiimide followed by reduction of the resulting amide with lithium aluminium hydride or alane. The reaction can also be performed by a standard one-pot procedure. Aldehydes or carboxylic acids of formula (IV/X) can be prepared analogously to the synthetic sequence described for alkylating reagents of formula (III/V) but by the use of acetal protected haloalkanal or the corresponding protected carboxylic acid derivatives.

The alkylation according to method 3), where Y is CO, CS, SO or SO₂ is conveniently performed by reacting the nitrogen anion of (VI) with (V). The nitrogen anion of (VI) can be prepared in an inert organic solvent, e.g. dimethyl formamide (DMF), dimethylsulfoxide (DMSO) or *N*-methylpyrrolidin-2-one (NMP), by the use of a strong base, e.g. NaH, before the alkylation.

The reduction of the double bond according to method 4) is generally performed by catalytic hydrogenation at low pressure (<3 atm.) in a Parr apparatus, or by using reducing agents such as diborane or hydroboric derivatives as produced *in situ* from NaBH₄ in trifluoroacetic acid in inert solvents such as tetrahydrofuran (THF), dioxane or diethyl ether. Starting materials of formula (VII) may be prepared by methods 1), 3), 7) and 8).

Reduction of amide groups according to methods 5) and 6) is most conveniently performed with lithium aluminium hydride or alane in an inert organic solvent such as e.g. tetrahydrofuran (THF) or diethylether from 0 °C to reflux temperature. Starting materials of formula (VIII) may be prepared by method 2), whereas starting materials of formula (IX) may be prepared by methods 1), 7) and 8).

The acylation according to method 8) is conveniently performed by the use of coupling reagents such as e.g. dicyclohexyl carbodiimide.

### Experimental Section

Melting points were determined on a Büchi B-540 apparatus and are uncorrected. Mass spectra were obtained on a Quattro MS-MS system from VG Biotech, Fisons Instruments. Analytical LC-MS data were obtained on a PE Sciex API 150EX instrument equipped with IonSpray source and Shimadzu LC-8A/SLC-10A LC system. The LC conditions (50 X 4.6 mm YMC ODS-A with 5 µm particle size) were linear gradient elution with water/acetonitrile/trifluoroacetic acid (90:10:0.05) to water/acetonitrile/trifluoroacetic acid (10:90:0.03) in 7 min at 2 mL/min. Purity was determined by integration of the UV trace (254 nm). The retention times Rₜ are expressed in minutes. ¹H NMR spectra were recorded at 500.13 MHz on a Bruker Avance DRX500 instrument or at 250.13 MHz on a Bruker AC 250 instrument. Deuterated chloroform (99.8%D) or dimethyl sulfoxide (99.9%D) were used as solvents. TMS was used as internal reference standard. Chemical shift values are expressed in ppm-values. The following abbreviations are used for multiplicity of NMR signals: s=singlet, d=doublet, t=triplet, q=quartet, qui=quintet, h=heptet, dd=double doublet, dt=double triplet, dq=double quartet, tt=triplet of triplets, m=multiplet, b=broad. NMR signals corresponding to acidic protons are generally omitted. Content of water in crystalline compounds was determined by Karl Fischer titration. For column chromatography silica gel of type Kieselgel 60, 40-60 mesh ASTM was used.

### Examples

### Preparation of intermediates

### A. Alkylating reagents

### 2-(3-Bromopropan-1-yl)-2H-naphth[1,8-cd]isothiazole 1,1-dioxide

A suspension of sodium hydride (4.1 g, 60% in mineral oil) and dimethyl formamide (250 mL) was kept at 20-23 °C followed by the addition of a solution of 2*H*-naphth[1,8-*cd*]isothiazole 1,1-dioxide (20 g) in dimethyl formamide (250 mL). The resulting mixture was stirred at room temperature for 10 min followed by the addition of a solution of 1,3-dibromopropane (40 mL) in dimethyl formamide (100 mL) at a temperature of 10-12 °C.

The resulting mixture was stirred at room temperature for 30 min and poured onto ice. The aqueous phase was extracted with ethyl acetate, and the combined organic phases were washed with brine, dried (MgSO₄) and concentrated *in vacuo*. The crude product was purified by flash chromatography on silicagel (eluent: ethyl acetate/heptane 1:4) to give the product as a crystalline compound (20.4 g).

### B. Amines

### 5-(Piperazin-1-yl)-1H-indole

A mixture of 5-nitro-1*H*-indole (34 g), palladium on activated carbon (Pd 5 %, water 50 %) (2.5 g) and ethyl acetate was shaken at room temperature for 1.5 h under 3 atmospheres of hydrogen. The mixture was filtered and the solvent was removed *in vacuo* to yield a crystalline compound (28 g), which was dissolved in tetrahydrofuran (400 mL). The solution was subsequently added to a boiling mixture of *N*-benzyliminodiacetic acid (54.4 g) and 1,1'-carbonyldiimidazole (82.4 g) in tetrahydrofuran (1100 mL), and the resulting mixture was boiled under reflux for 3 h. The mixture was filtered and concentrated *in vacuo.* The residue was purified by flash chromatography on silicagel (eluent: ethyl acetate/triethylamine 100:4) to give a white crystalline compound (57.5 g), which subsequently was dissolved in tetrahydrofuran (300 mL) and added to alane in tetrahydrofuran (500 mL) at 5-16 °C. The alane was prepared from lithium aluminium hydride (25 g) and concentrated sulphuric acid (32.3 g). The mixture was stirred at 5 °C for 45 min and subsequently quenched by the addition of water (50 mL), 15 % aqueous sodium hydroxide solution (25 mL) and water (125 mL). The mixture was dried (MgSO₄), filtered and concentrated *in vacuo*. The residue was purified by flash chromatography on silicagel (eluent: ethyl acetate) to give a brown oily compound (44.9 g), which subsequently was dissolved in methanol (1000 mL) and added ammonium formate (150 g) and palladium on activated carbon (Pd 5 %, water 50 %) (12 g). The mixture was boiled under reflux for 45 min, cooled, filtered and concentrated *in vacuo*. The residue was dissolved in tetrahydrofuran/ethyl acetate and added brine and concentrated aqueous ammonia solution under cooling to give a basic reaction mixture. The two phases were separated and the aqueous phase was extracted an additional two times with tetrahydrofuran/ethyl acetate. The combined organic phases were washed with brine, dried (MgSO₄) and concentrated *in* *vacuo*. The residue was crystallised from tetrahydrofuran/n-hetane to give the title compound (17.3 g).

### 6-(Piperazin-1-yl)-1H-indole

Prepared in a similar manner as 5-(piperazin-1-yl)-1*H*-indole starting from 6-amino-1*H*-indole (Brown et al. *J*. *Am*. *Chem*. *Soc*.. **1954**, *76*, 5149-5150).

### 5-(3,6-Dihydro-2H-pyridin-4-yl)-1H-indole

See WO 94/20459.

### 5-(Piperidin-4-yl)-1H-indole

A mixture of 5-(3,6-dihydro-2*H*-pyridin-4-yl)-1*H*-indole (3.4 g), platinum oxide (0.2 g) and acetic acid (50 mL) was shaken at room temperature for 24 h under 3 atmospheres of hydrogen. The mixture was filtered and the solvent was removed *in vacuo*. The residue was purified by flash chromatography on silicagel (eluent: 4 M ammonia in methanol) to give the title compound (1.3 g).

### Preparation of the compounds of the invention

### Example 1

### 1a, 2-{3-[4-(1H-Indol-5-yl)piperazin-1-yl]propan-1-yl}-2H-naphtho[1,8-cd]isothiazole 1,1-dioxide, hydrochloride

A mixture of 5-(piperazin-1-yl)-1*H*-indole (1.0 g), 2-(3-bromopropan-1-yl)-2*H*-naphth[1,8-*cd*]isothiazole 1,1-dioxide (1.6 g) and triethylamine (3.5 mL) in dimethyl formamide (10 mL) and butanone (100 mL) was boiled under reflux for 8 h. The mixture was filtered and concentrated *in vacuo*, and the residue was purified by flash chromatography on silicagel (eluent: ethyl acetate/triethylamine 100:4) to give the crude product as an yellow oil (2.1 g). The title compound was isolated as the hydrochloride salt from tetrahydrofuran as a white crystalline compound (1.6 g). Mp 275-278 °C. ¹H NMR (DMSO-d₆): 2.30-2.45 (m, 2H); 3.35-3.80 (m, 10H); 4.05 (t, 2H); 6.45 (s, 1H); 7.10 (d, 1H); 7.15 (d, 1H); 7.35 (s, 1H); 7.40-7.50 (m, 2H); 7.60-7.75 (m, 2H); 7.95 (t, 1H); 8.25 (d, 1H); 8.35 (d, 1H); 11.15 (s, 1H); 11.45 (broad s). MS m/z: 447 (MH+).

The following compounds were prepared in a similar manner:

### 1b, 2-{3-[4-(1H-Indol-6-yl)piperazin-1-yl]propan-1-yl}-2H-naphtho[1,8-cd]isothiazole 1,1-dioxide, hydrochloride

from 6-(piperazin-1-yl)-1*H*-indole and 2-(3-bromopropan-1-yl)-2*H*-naphth[1,8-*cd*]isothiazole 1,1-dioxide. Mp 214-215 °C. ¹H NMR (DMSO-d₆): 1.95-2.10 (m, 2H); 2.45-2.60 (m, 6H); 3.05-3.15 (m, 4H); 3.95 (t, 2H); 6.25 (s, 1H); 6.75 (d, 1H); 6.85 (s, 1H); 7.10-7.20 (m, 2H); 7.35 (d, 1H); 7.60 (d, 1H); 7.65 (t, 1H); 7.90 (t, 1H); 8.25 (d, 1H); 8.30 (d, 1H); 10.70 (s, 1H). MS m/z: 447 (MH+).

### 1c, 2-{3-[1-(1H-Indol-5-yl)-3,6-dihydro-2H-pyridin-4-yl]propan-1-yl}-2H-naphtho[1,8-cd]isothiazole 1,1-dioxide, hydrochloride

from 5-(3,6-dihydro-2*H*-pyridin-4-yl)-1*H*-indole and 2-(3-bromopropan-1-yl)-2*H*-naphth[1,8-*cd*]isothiazole 1,1-dioxide. ¹H NMR (DMSO-d₆):.2.00-2.10 (m, 2H); 2.55-2.65 (m, 4H); 2.65-2.70 (m, 2H); 3.10 (s, 2H); 3.95 (t, 2H); 6.10 (s, 1H); 6.40 (s, 1H); 7.15 (d, 1H); 7.25 (d, 1H); 7.30-7.40 (m, 2H); 7.60 (s, 1H); 7.65 (d, 1H); 7.70 (t, 1H); 7.90 (t, 1H); 8.25 (d, 1H); 8.30 (d, 1H); 11.05 (s, 1H). MS m/z: 444 (MH+).

### 1d, 2-{3-[1-(1H-Indol-5-yl)piperidin-4-yl]propan-1-yl}-2H-naphtho[1,8-cd]isothiazole 1,1-dioxide, hydrochloride

from 5-(piperidin-4-yl)-1*H*-indole and 2-(3-bromopropan-1-yl)-2*H*-naphth[1,8-*cd*]isothiazole 1,1-dioxide. LC/MS (m/z) 446 (MH+) 387; RT = 2.19; purity: 74%.

### Pharmacological Testing

The compounds of the invention were tested in well-recognised and reliable tests. The tests were as follows:

### Inhibition of the binding of [³H]YM-09151-2 to human dopamine D_{4.2} receptors

By this method, the inhibition by drugs of the binding of [³H]YM-09151-2 (0.06 nM) to membranes of human cloned dopamine D_{4.2} receptors expressed in CHO-cells is determined *in vitro*. Method modified from NEN Life Science Products, Inc., technical data certificate PC2533-10/96.

### Inhibition of the binding of [³H]Ketanserin to 5-HT_{2A} receptors

The compounds were tested with respect to their affinity for 5-HT_{2A} receptors by determining their ability to inhibit the binding of [³H]Ketanserin (0.50 nM) to membranes from rat brain (cortex) *in vitro.* Method described in Sánchez et al. *Drug Dev*. *Res*. **1991**, *22*, 239-250.

The test results are shown in table 1:

**Table 1:**

| Binding Data (IC₅₀ values in nM) or % inhibition of binding. | | |
|---|---|---|
| **Comp. No.** | **D**_{**4**}**-bind.** | **5-HT**_{**2A**}**-bind.** |
| **1a** | 1.9 | 0.79 |
| **1b** | 91 % (50 nM) | 1.8 |
| **1c** | 0.73 | 0.48 |
| **1d** | 83 % (50 nM) | 9.2 |

In general, the compounds of the invention have been found potently to inhibit the binding of [³H]YM-09151-2 to dopamine D₄ receptors. The compounds have also been tested in a functional assay described by Gazi et al. in *Br. J*. *Pharmacol*. **1999**, *128*, 613-620, and it was found that the compounds are antagonists or partial agonists at dopamine D₄ receptors.

The compounds have also been found to inhibit the binding of [³H]Ketanserin to 5-HT_{2A} receptors *in vitro*.

The compounds of the invention were also tested in the following tests:

### Inhibition of binding of [³H]Spiperone to rat dopamine D₂ receptors

By this method, the inhibition of drugs of the binding of [³H]Spiperone (0.5 nM) to dopamine D₂ receptors in membranes from rat corpus striatum is determined in vitro. Method and results as described in Hyttel et al. *J*. *Neurochem*. **1985**, *44*, 1615*-*1622.

### Inhibition of binding of [³H]Prazosine to rat alpha-1-receptors

By this method, the inhibition by drugs of the binding of [³H]Prazosin (0.25 nM) to alpha-1 receptors in membranes from rat brain is determined *in vitro*. Method modified from Hyttel et al. *J*. *Neurochem*. **1985**, *44*, 1615-1622.

The compounds have no substantial or only weak affinity for the dopamine D₂ receptor. Some of the compounds have no or only low affinity for the alpha-1 adrenergic receptor, which imply a low propensity to cause orthostatic hypotension, and no or only low sedative effect.

### Inhibition of the uptake of [³H]Serotonin into whole rat brain synaptosomes

The compounds were tested with respect to their 5-HT reuptake inhibiting effect by measuring their ability to inhibit the uptake of [³H]serotonin into whole rat brain synaptosomes *in vitro.* The assay was performed as described by Hyttel *Psychopharmacology* **1978**, *60*, 13.

Some compounds were shown to be 5-HT reuptake inhibitors. 5-HT reuptake inhibitors are well known antidepressant drugs.
Accordingly, the compounds of the invention are considered useful in the treatment of positive and negative symptoms of schizophrenia, other psychoses, anxiety disorders, such as generalised anxiety disorder, panic disorder and obsessive compulsive disorder, depression, aggression, cognitive disorders, side effects induced by conventional antipsychotic agents, migraine, attention deficit hyperactivity disorder and in the improvement of sleep. In particular, the compounds of the invention are considered useful in the treatment of positive and negative symptoms of schizophrenia without inducing extrapyramidal side effects.

### Formulation Examples

The pharmaceutical formulations of the invention may be prepared by conventional methods in the art.
For example: Tablets may be prepared by mixing the active ingredient with ordinary adjuvants and/or diluents and subsequently compressing the mixture in a conventional tabletting machine. Examples of adjuvants or diluents comprise: corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.
Solutions for injections may be prepared by dissolving the active ingredient and possible additives in a part of the solvent for injection, preferably sterile water, adjusting the solution to desired volume, sterilising the solution and filling it in suitable ampules or vials. Any suitable additive conventionally used in the art may be added, such as tonicity agents, preservatives, antioxidants, etc.
Typical examples of recipes for the formulation of the invention are as follows:
1) Tablets containing 5.0 mg of the active compound calculated as the free base:

| | |
|---|---|
| Active compound | 5.0 mg |
| Lactose | 60 mg |
| Maize starch | 30 mg |
| Hydroxypropylcellulose | 2.4 mg |
| Microcrystalline cellulose | 19.2 mg |
| Croscarmellose Sodium Type A | 2.4 mg |
| Magnesium stearate | 0.84 mg |

2) Tablets containing 0.5 mg of the active compound calculated as the free base:

| | |
|---|---|
| Active compound | 0.5 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Active compound | 25 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 ml |
| Flavour | 0.05 mg |
| Saccharin sodium | 0.5 mg |
| Water | ad 1 ml |

4) Solution for injection containing per millilitre:

| | |
|---|---|
| Active compound | 0.5 mg |
| Sorbitol | 5.1 mg |
| Acetic Acid | 0.05 mg |
| Saccharin sodium | 0.5 mg |
| Water | ad 1 ml |

## Claims

1. An indole derivative having the formula 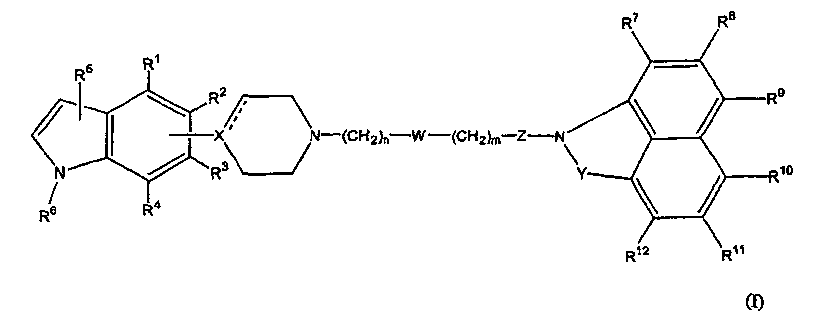 wherein Y is CO, CS, SO, SO₂ or CH₂; Z is CO, CS, SO, SO₂ or CH₂; provided that only one of Y and Z is CO, CS, SO or SO₂;
W is a bond, O, S, CO, CS, SO or SO₂;
n is 0-5, m is 0-5 and n + m is 1-6; provided that when W is O or S, then n ≥ 2 and m ≥ 1, when W is CO, CS, SO or SO₂, then n ≥ 1 and m ≥ 1;
X is N or CH and the dotted line represents no bond, or X is C and the dotted line represents a bond;
one of R¹, R², R³ and R⁴ forms a bond to X and the others of R¹, R², R³ and R⁴ and R⁵ and R⁷ - R¹² are selected form hydrogen, halogen, cyano, nitro, amino, C₁₋₆-alkyl-amino, di-( C₁-₆-alkyl )-amino, C₁₋₆-alkyl, C₂₋₆-alkenyl, C₂₋₆-alkynyl, C₁₋₆ alkoxy, C₁₋₆-alkylthio, hydroxy, C₁₋₆-alkyl substituted with hydroxy or thiol, C₃₋₈-cycloalkyl, C₃₋₈-cycloalkyl-C₁₋₆-alkyl, acyl, thioacyl, trifluoromethyl, trifluoromethylsulfonyl and C₁₋₆ alkylsulfonyl; and
R⁶ is hydrogen, C₁₋₆-alkyl, C₂₋₆-alkenyl, C₂₋₆-alkynyl, C₁₋₆-alkyl substituted with hydroxy or thiol, C₃₋₈-cycloalkyl, C₃₋₈-cycloalkyl-C₁₋₆-alkyl, acyl, thioacyl, trifluoromethylsulfonyl and C₁₋₆ alkylsulfonyl;
or pharmaceutically acceptable salts thereof.

2. A compound according to claim 1 wherein Z is CH₂ and Y is SO₂.

3. A compound according to claims 1 to 2 wherein one of R¹, R², R³ and R⁴ forms a bond to X and the others of R¹, R², R³ and R⁴ and R⁵ and R⁷ - R¹² are selected from hydrogen, halogen, cyano, nitro, amino, C₁₋₆-alkyl, C₁₋₆ alkoxy, C₁₋₆-alkylthio, hydroxy and trifluoromethyl, R⁶ is hydrogen, C₁₋₆-alkyl, C₁₋₆-alkylcarbonyl and W is a bond

4. A compound according to claims 1 to3 wherein R² or R³ form a bond to X.

5. A compound according to claims 1 to 4 wherein X is N.

6. A compound according to claims 1 to 4 wherein X is C.

7. A compound according to claims 1 to 4 wherein X is CH.

8. A compound according to claims 1 to 7 wherein W is a bond and n + m is 1 to 4.

9. A compound according to claim 1 which is selected from
2-{3-[4-(1H-Indol-5-yl)piperazin-1-yl]propan-1-yl}-2H-naphtho[1,8-cd]isothiazole 1,1-dioxide,
2-{3-[4-(1H-Indol-6-yl)piperazin-1-yl]propan-1-yl}-2H-naphtho[1,8-cd]isothiazole1,1-dioxide,
2-{3-[1-(1*H*-Indol-5-yl)-3,6-dihydro-2*H*-pyridin-4-yl]propan-1-yl}-2*H*-naphtho[1,8-cd]isothiazole 1,1-dioxide,
2-{3-[1-(1*H*-Indol-5-yl)piperidin-4-yl]propan-1-yl}-2*H*-naphtho[1,8-cd]isothiazole 1,1-dioxide
or a pharmaceutically acceptable acid addition salt thereof

10. A pharmaceutical composition **characterised in that** it comprises a compound of any of claims 1 to 9 in a therapeutically effective amount together with one or more pharmaceutically acceptable carriers or diluents.

11. Use of a compound of any of Claims 1 to 9 for the manufacture of a medicament useful in the treatment of positive and negative symptoms of schizophrenia, other psychoses, anxiety disorders, such as generalised anxiety disorder, panic disorder and obsessive compulsive disorder, depression, aggression, cognitive disorders, side effects induced by conventional antipsychotic agents, migraine, attention deficit hyperactivity disorder and in the improvement of sleep.

## Patentansprüche

1. Indol-Derivat mit der Formel: 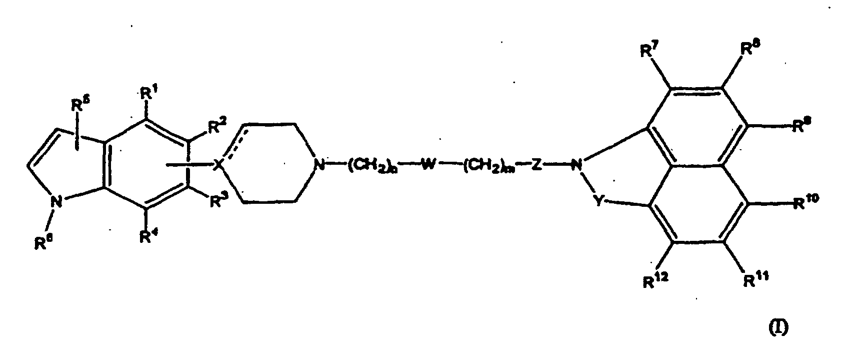 worin Y CO, CS, SO, SO₂ oder CH₂ ist; Z CO, CS, SO, SO₂ oder CH₂ ist; mit der Maßgabe, daß nur eines aus Y und Z CO, CS, SO oder SO₂ ist;
W eine Bindung, O, S, CO, CS, SO oder SO₂ ist;
n 0-5 ist, m 0-5 ist und n+m 1-6 ist; mit der Maßgabe, daß dann, wenn W O oder 5 ist, n ≥ 2 und m ≥ 1 ist, und daß dann, wenn W CO, CS, SO oder SO₂ ist, n ≥ 1 und m ≥ 1 ist;
X N oder CH ist und die gestrichelte Linie keine Bindung dargestellt oder X C ist und die gestrichelte Linie eine Bindung darstellt;
ein Vertreter aus R¹, R², R³ und R⁴ eine Bindung mit X bildet und die anderen aus R¹, R², R³ und R⁴ und R⁵ und R⁷-R¹² ausgewählt sind aus Wasserstoff, Halogen, Cyano, Nitro, Amino, C₁₋₆-Alkylamino, Di-(C₁₋₆-alkyl)-amino, C₁₋₆-Alkyl, C₂₋₆-Alkenyl, C₂₋₆-Alkinyl, C₁₋₆-Alkoxy, C₁₋₆-Alkylthio, Hydroxy, C₁₋₆-Alkyl, das mit Hydroxy oder Thiol substituiert ist, C₃₋₈-Cycloalkyl, C₃₋₈-Cycloalkyl-C₁₋₆-alkyl, Acyl, Thioacyl, Trifluormethyl, Trifluormethylsulfonyl und C₁₋₆-Alkylsulfonyl; und
R⁶ Wasserstoff, C₁₋₆-Alkyl, C₂₋₆-Alkenyl, C₂₋₆-Alkinyl, C₁₋₆-Alkyl, das mit Hydroxy oder Thiol substituiert ist, C₃₋₈-Cycloalkyl, C₃₋₈-Cycloalkyl-C₁₋₆-alkyl, Acyl, Thioacyl, Trifluormethylsulfonyl und C₁₋₆-Alkylsulfonyl ist;
oder pharmazeutisch akzeptable Salze davon.

2. Verbindung gemäß Anspruch 1, worin Z CH₂ ist und Y SO₂ ist.

3. Verbindung gemäß Ansprüchen 1 bis 2, worin ein Vertreter aus R¹, R², R³ und R⁴ und R⁵ und R⁷-R¹² ausgewählt sind aus Wasserstoff, Halogen, Cyano, Nitro, Amino, C₁₋₆-Alkyl, C₁₋₆-Alkoxy, C₁₋₆-Alkylthio, Hydroxy und Trifluormethyl, R⁶ Wasserstoff, C₁₋₆-Alkyl oder C₁₋₆-Alkylcarbonyl ist und W eine Bindung ist.

4. Verbindung gemäß Ansprüchen 1 bis 3, worin R² oder R³ eine Bindung mit X bilden.

5. Verbindung gemäß Ansprüchen 1 bis 4, worin X N ist.

6. Verbindung gemäß Ansprüchen 1 bis 4, worin X C ist.

7. Verbindung gemäß Ansprüchen 1 bis 4, worin X CH ist.

8. Verbindung gemäß Ansprüchen 1 bis 7, worin W eine Bindung ist n+m 1 bis 4 ist.

9. Verbindung gemäß Anspruch 1, die ausgewählt ist aus
2-{3-[4-(1H-Indol-5-yl)piperazin-1-yl]propan-1-yl}-2Hnaphtho[1,8-cd]isothiazol-1,1-dioxid,
2-{3-[4-(1H-Indol-6-yl)piperazin-1-yl]propan-1-yl}-2Hnaphtho[1,8-cd]isothiazol-1,1-dioxid,
2-[3-[1-(1H-Indol-5-yl)-3,6-dihydro-2H-pyridin-4-yl]propan-1-yl}-2H-naphtho[1,8-cd]isothiazol-1,1-dioxid,
2-{3-[1-(1H-Indol-5-yl)piperidin-4-yl]propan-1-yl}-2Hnaphtho[1,8-cd]isothiazol-1,1-dioxid
oder einem pharmazeutisch akzeptablen Säureadditionssalz davon.

10. Pharmazeutische Zusammensetzung, die **dadurch gekennzeichnet ist, daß** sie eine Verbindung gemäß einem der Ansprüche 1 bis 9 in einer therapeutisch wirksamen Menge zusammen mit einem oder mehreren pharmazeutisch akzeptablen Trägern oder Verdünnungsmitteln umfaßt.

11. Verwendung einer Verbindung gemäß einem der Ansprüche 1 bis 9 zur Herstellung eines Medikaments, das nützlich in der Behandlung von positiven und negativen Symptomen von Schizophrenie, anderen Psychosen, Angststörungen, wie allgemeine Angststörung, Panikstörung und Zwangsstörung, Depression, Aggression, kognitiven Störungen, durch herkömmliche Antipsychotika induzierten Nebenwirkungen, Migräne, Aufmerksamkeitsdefizit-Syndrom mit Hyperaktivität und in der Schlafverbesserung ist.

## Revendications

1. Dérivé d'indole de formule 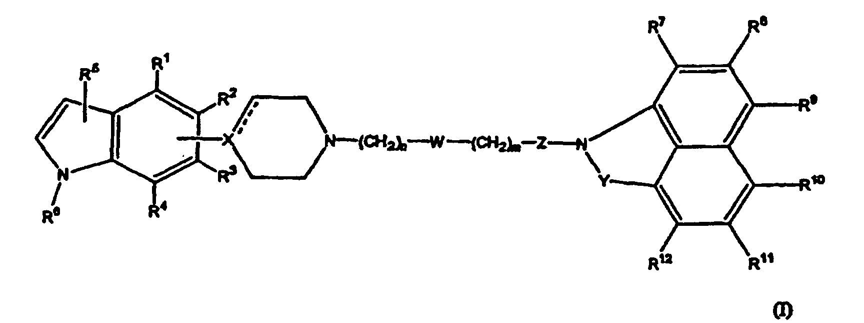 dans laquelle Y est CO, CS, SO, SO₂ ou CH₂; Z est CO, CS, SO, SO₂ ou CH₂; à condition que seul l'un des Y et Z soit CO, CS, SO ou SO₂;
W est une liaison, O, S, CO, CS, SO ou SO₂;
n est égal à 0-5, m est égal à 0-5 et n+m est égal à 1-6; à condition que, lorsque W est O ou S, n soit ≥ 2 et m ≥ 1, et que, lorsque W est CO, CS, SO, ou SO₂, n soit ≥ 1 et m ≥ 1;
X est N ou CH et la ligne pointillée ne représente pas de liaison, ou X est C et la ligne pointillée représente une liaison;
l'un des R¹, R², R³ et R⁴ forme une liaison avec X et les autres des R¹, R², R³ et R⁴, et R⁵ et R⁷-R¹² sont choisis parmi l'hydrogène, un halogène et les groupes cyano, nitro, amino, alkylamino en C₁-C₆, di(alkyl en C₁-C₆)amino, alkyle en C₁-C_{6,} alcényle en C₂-C₆, alcynyle en C₂-C₆, alcoxy en C₁-C₆, alkylthio en C₁-C₆, hydroxy, alkyle en C₁-C₆ substitué par hydroxy ou thiol, cycloalkyle en C₃-C₈, (cycloalkyl en C₃-C₈)-(alkyle en C₁-C₆), acyle, thioacyle, trifluorométhyle, trifluorométhylsulfonyle et alkylsulfonyle en C₁-C₆; et
R⁶ est l'hydrogène ou un groupe alkyle en C₁-C₆, alcényle en C₂-C₆, alcynyle en C₂-C₆, alkyle en C₁-C₆ substitué par hydroxy ou thiol, cycloalkyle en C₃-C₈, (cycloalkyl en C₃-C₈)-(alkyle en C₁-C₆), acyle, thioacyle, trifluorométhylsulfonyle et alkylsulfonyle en C₁-C₆;
ou leurs sels pharmaceutiquement acceptables.

2. Composé selon la revendication 1, dans lequel Z est CH₂ et Y est SO₂.

3. Composé selon les revendications 1 à 2, dans lequel l'un des R¹, R², R³ et R⁴ forme une liaison avec X et les autres des R¹, R², R³ et R⁴, et R⁵ et R⁷-R¹² sont choisis parmi l'hydrogène, un halogène et les groupes cyano, nitro, amino, alkyle en C₁-C₆, alcoxy en C₁-C₆, alkylthio en C₁-C₆, hydroxy et trifluorométhyle, R⁶ est l'hydrogène ou un groupe alkyle en C₁-C₆, (alkyl en C₁-C₆)carbonyle et W est une liaison.

4. Composé selon les revendications 1 à 3, dans lequel R² ou R³ forme une liaison avec X.

5. Composé selon les revendications 1 à 4, dans lequel X est N.

6. Composé selon les revendications 1 à 4, dans lequel X est C.

7. Composé selon les revendications 1 à 4, dans lequel X est CH.

8. Composé selon les revendications 1 à 7, dans lequel W est une liaison et n+m égale 1 à 4.

9. Composé selon la revendication 1, qui est choisi parmi
le 2-{3-[4-(1H-indol-5-yl)pipérazin-1-yl]propan-1-yl}-2H-naphto[1,8-cd]isothiazole-1,1-dioxyde,
le 2-{3-[4-(1H-indol-6-yl)pipérazin-1-yl]propan-1-yl}-2H-naphto[1,8-cd]isothiazole-1,1-dioxyde,
le 2-{3-[1-(1H-indol-5-yl)-3,6-dihydro-2H-pyridin-4-yl]propan-1-yl}-2H-naphto[1,8-cd]isothiazole-1,1-dioxyde,
le 2-{3-[1-(1H-indol-5-yl)pipéridin-4-yl]propan-1-yl}-2H-naphto[1,8-cd]isothiazole-1,1-dioxyde,
ou un de leurs sels d'addition d'acides pharmaceutiquement acceptables.

10. Composition pharmaceutique **caractérisée en ce qu'**elle comprend un composé selon l'une quelconque des revendications 1 à 9 en une quantité thérapeutiquement efficace avec un ou plusieurs supports ou diluants pharmaceutiquement acceptables.

11. Utilisation d'un composé selon l'une quelconque des revendications 1 à 9 pour la fabrication d'un médicament utile dans le traitement de symptômes positifs et négatifs de la schizophrénie, d'autres psychoses, de troubles d'anxiété comme l'anxiété généralisée, la panique et les troubles obsessionnels compulsifs, de la dépression, de l'agressivité, de troubles cognitifs, des effets secondaires induits par des agents antipsychotiques classiques, de la migraine, de l'hyperactivité avec déficit de l'attention, et pour améliorer le sommeil.
